Europäisches Patentamt

European Patent Office

Office européen des brevets

(11) Veröffentlichungsnummer: **0 339 260**
**A2**

## (12) EUROPÄISCHE PATENTANMELDUNG

(21) Anmeldenummer: **89105050.2**

(22) Anmeldetag: **21.03.89**

(51) Int. Cl.⁴: **A45D 34/04 , A45D 40/26**

(30) Priorität: **28.04.88 DE 3814305**

(43) Veröffentlichungstag der Anmeldung:
**02.11.89 Patentblatt 89/44**

(84) Benannte Vertragsstaaten:
**BE CH DE ES FR GB IT LI LU NL SE**

(71) Anmelder: **GEORG KARL GEKA-BRUSH GMBH**

**D-8809 Bechhofen-Waizendorf(DE)**

(72) Erfinder: **Fitjer, Holger**
**Lambrechtstrasse 15**
**D-8800 Ansbach(DE)**

(74) Vertreter: **Matschkur, Peter Dipl.-Phys.**
**Czowalla - Matschkur Patentanwälte Dr.**
**Kurt-Schumacher-Strasse 23**
**D-8500 Nürnberg 11(DE)**

(54) **Auftragsgerät für kosmetische und medizinische Anwendungen.**

(57) Auftragsgerät mit einem an einem Handgriff angeordneten vorzugsweise porösen Applikator zum Auftragen flüssiger oder pasteuser Massen, die aus einem Vorratsbehälter während des Auftrags nachfließen, insbesondere für kosmetische und medizinische Anwendungen, wobei der Vorratsbehälter und der Applikator ein lösbar am Handgriff gehaltertes Kopfbauteil für Einmalanwendung mit einer abgedichteten Vorratskammer bilden die mittels einer von außen betätigbaren vorzugsweise am Handgriff angeordneten Freigabeeinrichtung öffenbar ist.

FIG. 2

## Auftragsgerät für kosmetische und medizinische Anwendungen

Die Erfindung bezieht sich auf ein Auftragsgerät mit einem an einem Handgriff angeordneten vorzugsweise porösen Applikator zum Auftragen flüssiger oder pasteuser Massen, die aus einem Vorratsbehälter während des Auftrags nachfließen, insbesondere für kosmetische und medizinische Anwendungen.

Derartige Auftragsgeräte sind bereits in den verschiedenartigsten Auführungsformen bekanntgeworden, insbesondere beispielsweise als Rasierpinsel mit im Griffteil untergebrachtem Schaumspender oder aber entsprechende Zahnbürsten mit über den Stil und eine Bohrung zwischen den Borsten ausfließender Zahnpasta oder schließlich auch im Rahmen von Nagellackpinseln, bei denen der Nagellack aus der Flasche ebenfalls unmittelbar durch das Auf-den-Kopf-Stellen zwischen die Pinselborsten einläuft und somit auf die Fingernägel verteilt werden kann. Schließlich gibt es derartige Auftragsorgane seit einigen Jahren auch in großem Umfang als Schuhbürsten.

Bei all diesen Auftragsgeräten besteht nicht nur die Schwierigkeit einer Abdichtung des Vorratsgefäßes nach außen, insbesondere während der Nichtbenutzungszeiten. Darüber hinaus entsteht auch die erhebliche Schwierigkeit, daß beispielsweise bei medizinischen Anwendungen eine Wiederverwendbarkeit immer wieder des gleichen Applikators in Verbindung mit einem größeren Vorratsgefäß erhebliche gesundheitliche und hygienische Bedenken entgegenstehen.

Der Erfindung liegt daher die Aufgabe zugrunde ein Auftragsgerät der eingangs genannten Art so auszugestalten, daß es rasch sauber und einfach und ohne hygienische Bedenken auch bei Anwendungen für medizinische Zwecke für eine Flüssigkeits- oder Pastenauftragung geeignet ist.

Zur Lösung dieser Aufgabe ist erfindungsgemäß vorgesehen, daß der Vorratsbehälter und der Applikator ein lösbar am Handgriff gehaltertes Kopfbauteil, vorzugsweise für Einmalanwendung mit einer abgedichteten Vorratskammer bilden, die mittels einer von außen betätigbaren, insbesondere am Handgriff angeordneten Freigabeeinrichtung öffenbar ist.

Durch das erfindungsgemäße Kopfbauteil, welches nur einen relativ geringen Vorrat für eine einmalig Anwendung aufnimmt, ist sowohl die Gefahr beseitigt, daß die Flüssigkeit oder Paste während der Wartezeit bis zu einer erneuten Anwendung Schaden erleidet, eintrocknet oder sonstwie unbrauchbar wird. Darüber hinaus hat man auf diese Weise auch die höchstmögliche Garantie für hygienischen Auftrag, so daß selbst die Behandlung ansteckender Hautkrankheiten od.dgl. auf diese Art und Weise problemlos möglich ist, da ja das Kopfbauteil nach der einmaligen Anwendung weggeworfen und bei erneutem Bedarf durch ein neues Kopfbauteil ersetzt wird. Das Vorsehen einer Reihe von solchen Kopfbauteilen gegenüber einem größeren Vorratsgefäß im Handgriff wie es bisher üblich war, ist dabei für die Anwendung hochwertiger Flüssigkeiten oder Pasten, insbesondere aber für jede medizinische Anwendung, auch kaum teurer oder praktisch unersetzbar, da im anderen Fall ja ein Großteil der aufzutragenden Masse letztendlich sowieso eintrocknet oder sonstwie unbrauchbar wird oder aber da bei medizinscher Anwendung eine solche Mehrfachbenutzung von vorneherein aus hygienischen Gründen ausscheiden muß. Hinzu kommt schließlich noch, daß selbst bei großen Vorratsgefäßen häufig die Art der aufzutragenden Masse die Wiederverwendung des Applikators verhindert, so daß in jedem Fall zumindest der Applikator ausgetauscht werden müßte. Die Serienfertigung der erfindungsgemäßen Kopfbauteile mit einem quasi vergrößerten Steckabschnitt des Applikators, dessen Hohlraum die für eine Einmalanwendung notwendig Flüssigkeit oder Pastenmenge aufnimmt, ist auch nicht erheblich teurer als die Fertigung der Applikatoren selbst, wobei aber die Vorteile der erfindungsgemäßen Wegwerfeinheit die eventuellen Mehrkosten bei weitem wettmachen.

Dabei könnte gegebenenfalls vorgesehen sein, daß im Applikator eine Öffnung vorgesehen ist, die durch einen Stift verschlossen ist, der auf der gegenüberliegenden Seite in einem Handgriff übersteht, so daß er nur ein Stück zurückgezogen zu werden braucht, um die vorher abgedichtet im Inneren des Kopfbauteils untergebrachte Anwendungsmassen ausfließen zu lassen. Mit besonderem Vorteil kann aber in weiterer Ausbildung der Erfindung vorgesehen sein, daß der Applikator durch seine Dichtfolie vom Vorratsbehälter getrennt ist, die durch einen längsverschieblich im Handgriff gelagerten Dorn durchbohrbar ist, wobei das Kopfbauteil auf der am Haltegriff zu befestigenden Rückseite eine vom Dorn zu durchsetzende Abdecköffnung aufweist.

Nach dem Aufstecken eines neuen, noch abgedichteten Kopfbauteils auf den Handgriff wird unmittelbar vor der Anwendung der, bevorzugt mit einer längsverschiebbar am Haltegriff gelagerten Schieberplatte verbundene, Dorn nach vorne verschoben, wobei er zunächst die Abdichtöffnung auf der Rückseite des Kopfbauteils und dann schließlich auf der Vorderseite unmittelbar hinter dem eigentlichen Applikator auf die Dichtfolie trifft und diese perforiert. Die Flüssigkeit kann damit in den Applikator eindringen und über ihn auf der Behandlungsstelle verteilt werden. Nach der Behandlung wird das Kopfbauteil, das bevorzugt mit Rastsitz auf dem Haltgriff aufsteckbar ist, abgezogen, weggeworfen und im erneuten Behandlungsfall durch ein neues, unberührtes Kopfbauteil ersetzt.

In Ausgestaltung der Erfindung kann dabei vorgesehen sein, daß der Dorn bzw. der Schieber unter der Wirkung einer Rückholfeder steht, die ihn in seine zurückgezogene Stellung verspannt, so daß nicht durch geringe Unvorsichtigkeit eines neuen Kopfbauteils bereits eine Zerstörung der Dichtungen oder der Dichtfolie stattfindet und somit vorzeitig ein Ausfließen der Behandlungsmasse stattfinden kann. Dabei kann weiter vorgesehen sein, daß am vorderen Ende des Haltegriff eine längliche Führungsbohrung vorgesehen ist, in die der Dorn in seiner extremen Rückzugstellung unter der Wirkung der Feder einragt, so daß bei einem Vorschieben der Schieberplatte auch sichergestellt ist, daß der Dorn ohne zu verhaken in die Abdichtöffnung auf der Rückseite des Kopfbauteils eindringt um anschließend dann auf der Vorderseite die Dichtfolie zum Applikator zu durchdringen.

Eine in der Handhabung besonders geeignete Ausgestaltung eines erfindungsgemäßen Auftragsgeräts ergibt sich in Weiterbildung der Erfindung dadurch, daß das Kopfbauteil eine zur Befestigungsfläche geneigte Vorderfläche aufweist, die von einem als Schaumstoffplatte ausgebildeten Applikator belegt ist. Ein derartiger plattenförmiger Schaumstoffapplikator ermöglicht eine sehr gute gleichmäßige Verteilung der Behandlungsmasse, wobei durch die genannten Neigungen der Flächen zueinander zusätzlich erreicht wird, daß die Ebene des Applikators gegenüber der Längsachse des Haltegriffs geneigt ist und somit die Handhabung besonders vereinfacht ist.

Schließlich liegt es auch noch im Rahmen der Erfindung, das Kopfbauteil so auszubilden, daß es sich nach vorne zum Applikator hin konisch erweitert. Dadurch ergibt sich einerseits eine großflächige Applikatorfläche in Verbindung mit dem für die Handhabung güngstigen dünnen Haltegriff und gleichzeitig ein ausreichend großes Vorratsvolumen für die Behandlungsmasse.

Weitere Vorteile, Merkmale und Einzelheiten der Erfindung ergeben sich aus der nachfolgenden Beschreibung eines Ausführungsbeispiels sowie anhand der Zeichnung.

Dabei zeigen:

Fig. 1 eine perspektivische Ansicht eines erfindungsgemäßen Auftragsgeräts,

Fig. 2 einen Längsschnitt durch das Auftragsgerät nach Fig. 1,

Fig. 3 einen vergrößerten Teilschnitt durch den Übergangsbereich zwischen dem Haltegriff und dem lösbar aufgesteckten, für eine Einmalanwendung dienenden Kopfbauteil,

Fig. 4 einen vergrößerten Querschnitt längs der Linie IV-IV in Fig. 2 und

Fig. 5 einen vergrößerten Schnitt etwa längs der Linie V-V in Fig. 2.

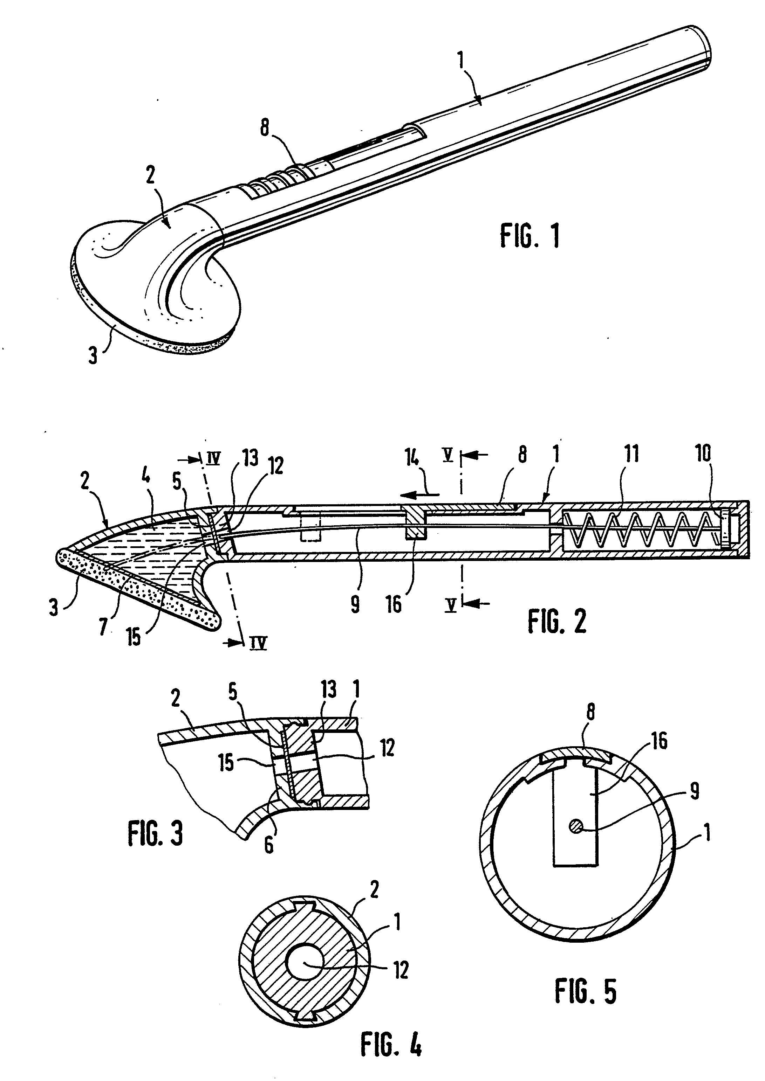

Das erfindungsgemäße Auftragsgerät besteht aus einem als langgestreckter Stil ausgebildeten Handgriff 1 und einem lösbar mit Rastsitz daran ansteckbaren Kopfbauteil 2 mit einem im dargestellten Ausführungsbeispiel als ebene Schaumstoffplatte ausgebildeten Applikator 3. Das Kopfbauteil 2 enthält außer dem Applikator 3 eine als Vorratsgefäß dienenden Hohlraum 4 zur Aufnahme einer flüssigen oder pastösen Masse, die mit Hilfe des Applikators 3 bei kosmetischen oder medizinischen Anwendungen aufgetragen werden soll, wobei die Masse lediglich für eine Einmalaufwendung ausgelegt ist. Zu diesem Zweck ist der Innenraum 4 des Kopfbauteils 2 abgedichtet, was zum einen mit Hilfe einer Abdichtfolie 5 auf der Außenseite der im Haltegriff 1 anliegenden Rückwand 6 erfolgt und zum anderen durch eine hinter dem Applikator 3 vorgesehen Folie 7. Zur Öffnung des Vorratsbehälters und zum Austragen der Flüssigkeit aus dem Innenraum 4 über den Applikator 3 dient ein mit einer am Handgriff 1 verschiebbar gelagerten Schieberplatte 8 verbundener Dorn 9 der durch eine am inneren Ende angeordneten Platte 10 unter der Wirkung einer Rückholfeder 11 steht. In der in Fig. 2 gezeigten zurückgezogenen Stellung des Dorns ragt sein vorderes Ende in eine Längsbohrung 12 der Vorderwand 13 des Handgriffs 1 ein und wird dadurch zwangsweise so geführt, daß beim Nach-vorne-Schieben der Schieberplatte 8 in Richtung des Pfeils 14 der Dorn 9 zunächst die Dichtfolie 5 durchsetzt um dann über eine Bohrung 15 ins Innere der Vorratskammer 4 zu gelangen. Schließlich trifft er - wie in Fig. 2 strichpunktiert darge stellt ist - auf die Dichtfolie 7 und perforiert diese, so daß flüssige oder pastöse Masse im Inneren des Kopfbauteils 2 durch diese Perforierung in den Applikator 3 austreten und aufgrund dessen Porosität nach außen gelangen und schließlich mit seiner Hilfe an der jeweiligen Behandlungsstelle verteilt werden kann. Nach Entleerung des Inhalts des Kopfbauteils, die so ausgerichtet ist, daß sie gerade für eine Einmalanwendung geeignet ist, wird das Kopfbauteil 2, welches durch eine Schnapp-Rastverbindung (vgl. Fig. 3) am Handgriff 1 gehaltert ist, abgezogen und im erneuten Anwendungsfall durch ein neues Kopfbauteil 2 ersetzt. Dabei ist die Ausgestaltung so getroffen, daß zwangsweise durch Nuten und Federn (vgl. Fig. 4) eine bestimmte Zuordnung des Kopfbauteils 2 zum Handgriff beim Aufstecken erzwungen wird.

In Fig. 5 erkennt man besonders deutlich den Mitnehmerzapfen 16 der Schieberplatte 8, in welcher der Dorn 9 gehaltert ist.

Durch die Neigung der Ebene des Applikators 3 gegenüber der Ebene der Rückwand 6 des Kopfbauteils 2 ergibt sich eine abgewinkelte Ausbildung des Auftragsgeräts, welches eine besonders gute Handhabbarkeit gewährleistet.

Die Erfindung ist nicht auf das dargestellte Ausführungsbeispiel beschränkt. Ersichtlich ließen sich eine Reihe von anderen Ausgestaltungen zur Freigabe des flüssigen oder pastösen Inhalts des Kopfbauteils im Bedarfsfall vorsehen. Entscheidend ist, daß in dem als Wegwerfeinheit ausgebildeten Kopfbauteil mit einem Applikator gleichzeitig die Auftragsmenge für eine Einmalanwendung untergebracht ist und daß erst im Bedarfsfall die Masse durch Freigabe einer Öffnung nach außen austreten kann.

## Ansprüche

1. Auftragsgerät mit einem an einem Handgriff angeordneten vorzugsweise porösen Applikator zum Auftragen flüssiger oder pasteuser Massen, die aus einem Vorratsbehälter während des Auftrags nachfließen, insbesondere für kosmetische und medizinische Anwendungen, dadurch gekennzeichnet, daß der Vorratsbehälter (4) und der Applikator (3) ein lösbar am Handgriff (1) gehaltertes Kopfbauteil (2) für Einmalanwendung mit einer abgedichteten Vorratskammer (4) bilden die mittels einer von außen betätigbaren vorzugsweise am Handgriff (1) angeordneten Freigabeeinrichtung öffenbar ist.

2. Auftragsgerät nach Anspruch 1, dadurch gekennzeichnet, daß der Applikator (3) durch eine Dichtfolie (7) von der Vorratskammer (4) getrennt ist, die durch einen längsverschieblich im Handgriff (1) gelagerten Dorn (9) durchbohrbar ist, wobei das Kopfbauteil (2) in der am Haltegriff (1) zu befestigenden Rückwand (6) eine vom Dorn (9) zu durchsetzende Abdichtöffnung ausweist.

3. Auftragsgerät nach Anspruch 2, dadurch gekennzeichnet, daß der Dorn (9) mit einer längsverschiebbar am Haltegriff (1) gelagerten Schieberplatte (8) verbunden ist.

4. Auftragsgerät nach Anspruch 2 oder 3, dadurch gekennzeichnet, daß der Dorn (9) bzw. die Schieberplatte (8) unter der Wirkung einer Rückholfeder (11) steht.

5. Auftragsgerät nach einem der Ansprüche 1 bis 4, gekennzeichnet durch eine Führungsbohrung (12) am vorderen Ende des Haltegriffs (1) in die der Dorn (9) in seiner Rückzugstellung einragt.

6. Auftragsgerät nach einem der Ansprüche 1 bis 5, dadurch gekennzeichnet, daß das Kopfbauteil (2) eine zur Rückwand (6) geneigte Vorderfläche aufweist, die von einem als Schaumstoffplatte ausgebildeten Applikator 3 belegt ist.

7. Auftragsgerät nach einem der Ansprüche 1 bis 6, dadurch gekennzeichnet, daß sich das Kopfbauteil (2) nach vorne zum Applikator (3) hin konisch erweitert.

8. Auftragsgerät nach einem der Ansprüche 1 bis 7, dadurch gekennzeichnet, daß das Kopfbauteil (2) mit Rastsitz auf den Haltegriff (1) aufsteckbar ist.

FIG. 1

FIG. 2

FIG. 3

FIG. 4

FIG. 5